# EUROPÄISCHE PATENTSCHRIFT

(11) **EP 1 404 341 B1**
(45) Veröffentlichungstag und Bekanntmachung des Hinweises auf die Patenterteilung: **27.09.2006**
(21) Anmeldenummer: 02751112.0
(22) Anmeldetag: 05.07.2002
(51) Int. Cl.: A61K 31/55, A61K 31/517, A61K 31/485, A61P 25/34

(54) **WIRKSTOFF-KOMBINATION ZUR MEDIKAMENTÖSEN THERAPIE DER NIKOTINABHÄNGIGKEIT**
ACTIVE SUBSTANCE COMBINATION FOR MEDICAMENTOUS THERAPY OF NICOTINE DEPENDENCY
COMBINAISON DE SUBSTANCES ACTIVES DESTINEE AU TRAITEMENT MEDICAMENTEUX DE LA DEPENDANCE A LA NICOTINE

(30) Priorität: 12.07.2001 DE 10134038
(43) Veröffentlichungstag der Anmeldung: 07.04.2004
(73) Patentinhaber: HF ARZNEIMITTELFORSCHUNG GmbH, 59368 Werne (DE)
(72) Erfinder: MOORMANN, Joachim, 59368 Werne (DE); MUCKE, Hermann, A-1160 Wien (AT); OPITZ, Klaus, 48157 Münster (DE)
(74) Vertreter: Flaccus, Rolf-Dieter
(86) Internationale Anmeldenummer: PCT/EP2002/007477
(87) Internationale Veröffentlichungsnummer: WO 2003/007966

(56) Entgegenhaltungen:
- WO-A-00/38686
- WO-A-00/48445
- WO-A-01/12196
- WO-A-01/43697
- WO-A-94/16708
- WO-A-97/33581
- WO-A-99/03347
- US-A- 4 573 995

## Beschreibung

Die vorliegende Erfindung betrifft Wirkstoff-Kombinationen und deren Verwendung zur medikamentösen Therapie der Nikotinabhängigkeit, insbesondere in Bezug auf den Zigarettenkonsum. Dabei besteht die Wirkstoff-Kombination aus zumindest einem Modulator des cholinergen Systems mit zumindest einer das Opioid-Rezeptorsystem modulierenden Substanz. Des weiteren betrifft die vorliegende Erfindung die Verwendung der angesprochenen Wirkstoff-Kombinationen zur Herstellung von Arzneimitteln, die zur Therapie des Nikotinkonsums, insbesondere des Konsums von Zigaretten, beitragen.

Die Zuführung von Nikotin und verwandten Tabakalkaloiden durch inhalierendes Rauchen, weit seltener auch durch Kauen oder Schnupfen von Tabakprodukten, hat zentralnervös anregende Effekte, die vor allem auf der Stimulierung der cholinergen und dopaminergen Reizleitungssysteme beruhen. Nach heutigem Erkenntnisstand ist dies auf eine funktionelle Aktivierung und verstärkte Expression der präsynaptischen "nikotinischen" Acetylcholin-Rezeptoren (nAChR) zurückzuführen, an denen außer dem natürlichen Agonisten Acetylcholin auch Nikotin in gleicher Weise wirkt und die vermehrte Ausschüttung der betreffenden Neurotransmitter (Acetylcholin und Dopamin) bewirken kann. In gleicher Weise, nämlich in Richtung auf eine Erhöhung des intrasynaptischen Dopaminspiegels, wirkt sich die Hemmung der Monoaminooxidase (MAO)-Enzyme durch Tabakprodukte aus; starke Raucher weisen eine um 20-40% erniedrigte Aktivität von MAO-A und MAO-B auf (siehe z.B. Berlin und Anthenelli, *Int. J. Neuropsychopharmacal.* 2001; 4(1):33-42). Das Zusammenspiel dieser cholinergen und dopaminergen Faktoren bewirkt einen erheblichen Teil der vom Raucher erwünschten kognitiven und stimmungsaufhellenden "Rewarding"-Effekte (siehe dazu Volodymyr et al., *Nature* 1997; Vol. 390, 401-404), jedoch werden auch andere Reizleitungssysteme, welche Noradrenalin, Serotonin, Gamma-Amino-buttersäure (GABA) und insbesondere auch opioidähnlich wirkende Peptide als Neurotransmitter verwenden, direkt oder indirekt moduliert und in ein neues, gegenüber dem Nichtraucher verändertes neurobiologisches Gleichgewicht gebracht.

Starkes und über Jahre fortgesetztes Rauchen führt bekanntermaßen zu einer Fülle schwerer und mit erheblicher Mortalität verbundener funktioneller Erkrankungen der Lungen und des Herz-Kreislauf-Systems sowie zu einem vermehrten Auftreten bestimmter Karzinome. In Nationen mit entwickelten Gesundheitssystemen ist das Rauchen heute die häufigste Ursache von statistisch vorzeitigen Todesfällen. Für Deutschland rechnet man mit 11.0000 direkt nikotinbedingten Todesfällen und 80 Milliarden Mark jährlich an Folgekosten.

Aufgrund der oben erwähnten nikotininduzierten neurophysiologischen Veränderungen bewirkt jedoch der Versuch, das Rauchen einzuschränken oder einzustellen, erhebliche Entzugserscheinungen. Tatsächlich liegt der Erfolg der Behandlung der Nikotinabhängigkeit mit permanenten Abstinenzraten zwischen 10 und 35 Prozent insgesamt noch unter den Resultaten bei Alkoholabhängigkeit. Ärztliches Zureden allein ist nur in ca. 5% der Fälle erfolgreich. Pharmakologische Substitution durch Nikotinpflaster hat dauernde Erfolgsquoten von 10-15%, was sich durch zusätzliche Verhaltenstherapie im besten Fall auf 30-35% steigern lässt. Dabei ist jedoch zu bedenken, dass die transdermale Verabreichung von Nikotin zwar die Aufnahme von Karzinogenen aus dem Rauch ausschaltet, die direkt nikotinbedingten kardiovaskulären Risiken jedoch in keiner Weise beeinflusst.

Eine nikotinfreie orale Substitution steht in Form des Wirkstoffes Bupropion (Zyban®, GlaxoSmithKline) zur Verfügung, der auf noradrenerger und dopaminerger Ebene wirkt und in klinischen Studien eine 1-Jahres-Abstinenzquote von 28% (verglichen mit 8% für Placebo) erzielt hat und auch in anderen Parametern der Rauchentwöhnung nicht signifikant wirkungsvoller als transdermales Nikotin ist.

Es besteht somit in unveränderter Weise Bedarf an einer nicht auf direkter Nikotinsubstitution beruhenden Therapie, welche die Reduktion des Tabakkonsums in unschädlicher und möglichst nebenwirkungsarmer Weise unterstützt. Es fehlt daher seit Jahren nicht an versuchen, pharmakologische Verbesserungen in die Raucherentwöhnung einzuführen, wobei neben cholinergen Modulatoren den Opioidantagonisten besondere Aufmerksamkeit geschenkt wird, da die das Substanzverlangen ("Craving") bewirkenden Mechanismen im Opioid-System lokalisiert gesehen werden.

Als Alternative zur Unterstützung des Nikotinentzuges mittels cholinerger Modulatoren wird z.B. in den Druckschriften DE 43 01 782 (äquivalent EP 0 680 326 bzw. US 5 643 905) die Verwendung von Galanthamin vorgeschlagen, welches das Verlangen nach Nikotin unterdrücken soll. In gleicher Weise gilt dies für Desoxypeganin, welches in DE 199 06 979 (äquivalent WO 00 48 445) für diesen Zweck beansprucht wird und aufgrund seiner gleichzeitigen Hemmung von Monoaminooxidasen besonders hohes therapeutisches Potential hat.

Darüberhinaus beschreibt US 5 932 238 ein für Galanthamin geeignetes transdermales therapeutisches System.

Galanthamin wird auch zur Behandlung der Poliomyelitis, der Alzheimerschen Krankheit und verschiedener Erkrankungen des Nervensystems eingesetzt, sowie zur Behandlung des Engwinkelglaukoms.

Galanthamin bzw. Galantamin (4a,5,9,10,11,12-Hexahydro-3-methoxy-11-methyl-6-H-benzofuro-(3a,3,2-ef)-(2)-benzazepin-6-ol) ist ein tetracyclisches Alkaloid, das bei bestimmten Pflanzen, insbesondere bei Amaryllidaceen, vorkommt. Es kann aus diesen Pflanzen mittels bekannter Verfahren gewonnen werden (z. B. gemäß DE 195 09 663 A1 oder DE-PS 11 93 061) oder auf synthetischem Wege (z. B. Kametani et al., *Chem. Soc. C.* 6, 1043-1047 (1971); Shimizu et al., *Heterocycles* 8, 277-282 (1977)) hergestellt werden.

Aufgrund seiner pharmakologischen Eigenschaften wird Galanthamin zur Gruppe der reversibel wirkenden Cholinesterasehemmstoffe gezählt. Gleichzeitig stimuliert Galanthamin auch die Ausschüttung des Neurotransmitters Acetylcholin durch direkte Stimulation der präsynaptischen nikotinischen Acetylcholinrezeptoren. Ein analoger Vorgang erfolgt auch auf dopaminergen präsynaptischen Nervenendigungen, wo es die Ausschüttung von Dopamin fördert. Diese Eigenschaften des Galanthamin sollen nach gängigen Theorien das "Craving" unabhängig von kognitiver Kontrolle reduzieren. Dies stellt den theoretischen Unterbau der auf die Therapie der Alkoholabhängigkeit bzw. der Symptome des Alkoholentzuges Bezug nehmenden Druckschriften DE 40 10 079 und US 5 932 238 dar und findet auch in der Patentschrift DE 101 29 265.1, welche Kombinationen von Galanthamin mit Hemmstoffen neuroexzitatorischer Prozesse im Alkoholabusus beschreibt, entsprechende Erwähnung.

Der für Galanthamin beschriebene kombinierte direkte cholinerge und indirekte dopaminerge Effekt läßt sich auch mit Substanzen erzielen, die gleichzeitig die Acetylcholinesterase und die Monoaminoxidase inhibieren. Dies ist beispielsweise beim Desoxypeganin der Fall, das insbesondere in der älteren Literatur auch als Desoxyvasicin bezeichnet wird. Darüberhinaus wurde vorgeschlagen, Desoxypeganin ebenfalls zur Behandlung der Nikotinabhängigkeit durch Verminderung des Verlangens nach Nikotin oder zur Substitutionstherapie von Drogensüchtigen bzw. zur Behandlung von Entzugserscheinungen während einer Entzugstherapie anzuwenden (WO 00 48 582) sowie auch zur medikamentösen Therapie des Alkoholmißbrauchs und der Alzheimer'schen Demenz. Außerdem kann Desoxypeganin als Cholinesterasehemmer als Antidot oder Prophylaktikum bei Vergiftungen durch organische Phosphorsäureester eingesetzt werden, wobei es die zerebrale Wirkung cholinerger Gifte antagonisiert.

Desoxypeganin (1,2,3,9-Tetrahydropyrrolo[2,1-b]chinazolin) ist ein Alkaloid der Summenformel C₁₁H₁₂N₂, das in Pflanzen aus der Familie der Zygophyllaceae enthalten ist. Die Gewinnung von Desoxypeganin erfolgt vorzugsweise durch Isolierung aus der Steppenraute (*Peganum harmala*) oder durch Synthese.

Trotz ihrer doppelten Wirkmechanismen sind Galanthamin und Desoxypeganin nur beschränkt geeignet, das Rauchverlangen wirksam zu unterdrücken. Der Grund dafür dürfte darin liegen, daß das Verlangen nach Tabakkonsum nach derzeitigem Wissensstand von dem durch regelmäßiges Rauchen aktivierten endogenen Opioid-System entscheidend mitbestimmt wird.

Opioidrezeptor-Antagonisten, von denen einige in der Entzugstherapie für Alkohol- und Opiatabusus seit längerer Zeit in klinischer Verwendung stehen, sind daher ebenfalls zur Unterstützung der Rauchentwöhnung vorgeschlagen worden, so z.B. die eng verwandten Wirkstoffe Naltrexon, Naloxon und Nalbuphin in oralen Formulierungen bzw. in Form transdermaler therapeutischer Systeme (US 6 004 970, US 4 573 995), ebenso Nalmefen (US 5 852 032). Gleiches gilt für die 5,9-Dimethylbenzomorphane Cyclazocin (US-5 965 567, Maisonneuve und Glick, *NeuroReport* 1999; 10: 693-696) und Pentazocin. Diese zeigen ein differenziertes Wirkungsspektrum (an mu-Opioidrezeptoren antagonistisch und an kappa-Opioidrezeptoren agonistisch, ebenso Modulation von sigma-Rezeptoren).

Da von diesen Substanzen über Naltrexon die umfangreichsten Daten bei der Anwendung am Menschen vorliegen, wurde es auch zur Bekämpfung des Rauchverlangens am intensivsten untersucht. Die dabei erhaltenen Ergebnisse sind durchaus widersprüchlich. Obwohl Fallberichte und kleinere Studien zeigten, dass Naltrexon unter bestimmten Umständen den Rauchgenuß und die Zahl der pro Tag gerauchten Zigaretten reduzieren kann *(Psychopharmacology* 1998; 140(2): 185-190 sowie *J. Clin. Psychiatry* 1998; 59(1): 30-31 und *Pharmacol. Biochem. Behav.* 2000; 66(3): 563-572), waren die Resultate dreier randomisierter klinischer Studien mit insgesamt 180 Rauchern negativ *(Psychopharmacology* 1995; 120(4): 418-425, Addiction 1999; 94(8): 1227-1237 und *J*. *Addict. Dis.* 1999; 18(1): 31-40). Ebenso stehen Daten, nach denen Naltrexon in Kombination mit transdermal verabreichtem Nikotin den Rauchstimulus dämpfen soll (*Psychopharmacology* 1999; 142(2): 139-143), andere Ergebnisse gegenüber, nach denen Naltrexon die tabakvermeidende Wirkung zuvor applizierter Nikotinpflaster sogar aufhebt (*Psychopharmacology* 1999; 143(4): 339-346), was im Einklang mit Daten aus früheren Tierversuchen steht. Erkenntnisse aus elektrophysiologischen in vitro-Experimenten, die nahe legen, dass Naltrexon bestimmte Subtypen nikotinischer Rezeptoren im Gehirn hinsichtlich Aktivität und Expression in unterschiedlicher Weise beeinflusst (*Neuropharmacology* 2000; 39(13), 2740-2755), können eventuell als teilweise Erklärung des letztgenannten Phänomens dienen, ebenso wie individuelle Unterschiede der Aufnahme von oral gegebenem Naltrexon und seiner im Gehirn erreichten Konzentration.

Insgesamt wird daher die Reduktion des Tabakkonsums also weder durch alleinige Gabe von Modulatoren nikotinischer Rezeptoren noch durch die alleinige Gabe von im Alkoholentzug eingesetzten Opioidrezeptor-Antagonisten in befriedigender Weise erreicht. Ziel der vorliegenden Erfindung war daher die Bereitstellung von Wirkstoffkombinationen zur Herstellung von Arzneimitteln, durch die das Rauchverlangen in besserer Weise als durch die oben geschilderten Verfahren gedämpft wird, ohne jedoch Nebenwirkungen hervorzurufen, die ihrerseits das durch vermehrten Streß hervorgerufene Rauchverlangen steigern.

Überraschenderweise wurde gefunden, daß die der vorliegenden Erfindung zugrundeliegende Aufgabe durch die Kombination von bestimmten als Modulatoren des cholinergen Systems wirkenden Substanzen mit primär als Opioidrezeptor-Antagonisten wirkenden Substanzen besonders gut gelöst werden kann.

Erfindungsgemäß werden Modulatoren des cholinergen Systems verwendet, die neben ihrer inhibitorischen Wirkung auf Cholinesterasen auch auf dopaminerge Nervenendigungen wirken. Dies kann beispielsweise durch Substanzen erfolgen, die als Cholinesteraseinhibitoren auch nikotinische Acetylcholinrezeptoren an den präsynaptischen Nervenendigungen cholinerger und domapinerger Nervenendigungen direkt stimulieren oder durch Substanzen, die gleichzeitig die Acetylcholinesterase und die Monoaminoxidase inhibieren.

Bevorzugt werden als Modulatoren des cholinergen Systems mit den zuvor angesprochenen Eigenschaften Galanthamin oder Desoxypeganin oder deren pharmakologisch akzeptable Derivate verwendet. Es versteht sich für den Fachmann von selbst, daß Galanthamin oder Desoxypeganin in Form ihrer freien Basen oder in Form ihrer bekannten Salze oder Derivate verwendet werden. So können beispielsweise anstelle der Salze bzw. Additionsverbindungen des Galanthamins auch sämtliche in der wissenschaftlichen Literatur und in Patentschriften aufgeführten bzw. beanspruchten Derivate des Galanthamins verwendet werden, insoweit diese entweder Inhibitoren der Cholinesterase-Enzyme oder Modulatoren der nikotinischen Acetylcholinrezeptoren sind bzw. beide pharmakologischen Aktivitäten kombiniert ausüben. Darunter sind insbesondere zu verstehen:
- Die in den Patentschriften der Familien WO-96 12 692 / EP 0 787 115 / US 6 043 359 sowie WO 97 40 049 / EP 0 897 387 und WO 0 32 199 (Waldheim Pharmazeutika GmbH. bzw. Sanochemia Pharmazeutika AG) angeführten Verbindungen, darunter insbesondere:
   (-)-N-Demethylgalanthamin;
   (-)-(N-Demethyl)-N-allylgalanthamin;
   (-)-(6-Demethoxy)-6-hydroxygalanthamin (SPH-1088);
   (+/-)N-Demethylgalanthamin N-tert-butyl carboxamid (SPH-1221);
   (-)N-Demethylgalanthamin N-tert-butyl carboxamid
- Die in den Patentschriften der Familien EP 0 648 771 und EP 0 653 427 (Hoechst Roussel Pharmaceuticals Inc.) sowie *Drugs Fut.* 21(6), 621-635 (1996) und *J. Pharmacol. Exp. Ther.* 277(2), 728-738 (1996) angeführten Verbindungen, darunter insbesondere:
   (-)-6-O-Demethylgalanthamin;
   (-)-(6-O-Acetyl)-6-O-demethylgalanthamin (P11012);
   (-)-(6-O-Demethyl)-6-O-[(adamantan-1-yl)carbonyl]galanthamin (P11149);
   (-)-(6-O-Demethyl)-6-O-(triethylsilyl)galanthamin;
   (-)-(6-O-Demethyl)-6-O-(triisopropylsilyl)galanthamin;
   (-)-(6-O-Demethyl)-6-O-(trimethylsilyl)galanthamin;
- Die in den Patentschriften der Familien WO 97 03 987 / EP 0 839 149 / US 5 958 903 (Societe de Conseils de Recherches et D'Applications Scientifiques, S.C.R.A.S) angeführten Verbindungen, darunter insbesondere:
   (6-O-Demethyl)-6-O-(8'-phtalimidooctyl)galanthaminium bromhydrat;
   (6-O-Demethyl)-6-O-(4'-phtalimidobutyl)galanthaminium bromhydrat;
   (6-O-Demethyl)-6-O-(10'-phtalimidodecyl)galanthaminium bromhydrat;
   (6-O-Demethyl)-6-O-(12'-phtalimidododecyl)galanthaminium bromhydrat;
   10-N-Demethyl-10-N-(10'-phtalimidobutyl)galanthaminium trifluoracetat;
   10-N-Demethyl-10-N-(10'-phtalimidohexyl)galanthaminium trifluoracetat
   10-N-Demethyl-10-N-(10'-phtalimidooctyl)galanthaminium bromhydrat;
   10-N-Demethyl-10-N-(10'-phtalimidododecyl)galanthaminium bromhydrat;
   10-N-Demethyl-10-N-(12'-phtalimidododecyl)galanthaminium bromhydrat;
   10-N-Demethyl-10-N-(6'-pyrollohexyl)galanthaminium bromhydrat

Die unter anderem in der Veröffentlichung *Bioorg. Med. Chem.* 6(10), 1835-1850 (1998) beschriebenen (-)N,N'-Demethyl-N,N'-Bis-Galantaminderivate folgender Strukturformel, wobei die Überbrückungsgruppe ("Alkyl-Spacer") zwischen den Stickstoffatomen der beiden Galanthamin-Moleküle 3-10 CH₂-Gruppen lang sein kann und unabhängig davon eines der beiden Galanthamin-Moleküle eine positive Ladung am Stickstoffatom tragen kann (Galanthaminium-Kation): 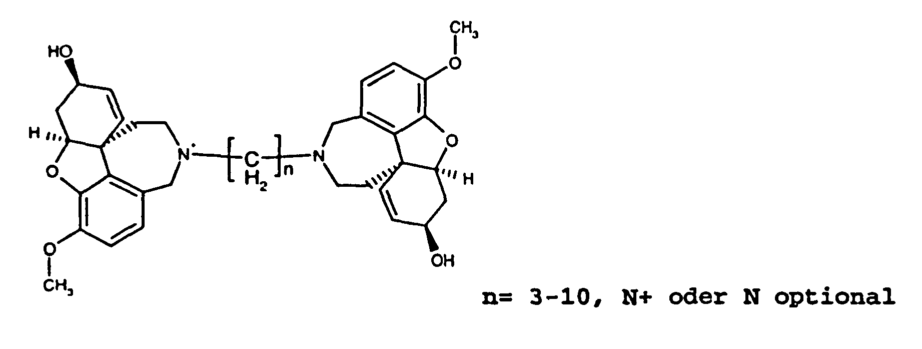
- Das unter anderem in der Veröffentlichung *J*. *Cerebral Blood Flow Metab.* 19(Suppl. 1), S19 (1999) sowie in Proteins 42, 182-191 (2001) beschriebene (-)N-Demethyl-N-(3-piperidinopropyl)galanthamin (SPH-1286) sowie dessen Analoga mit , Alkyl-Spacern" von bis zu 10 CH₂-Gruppen Länge:

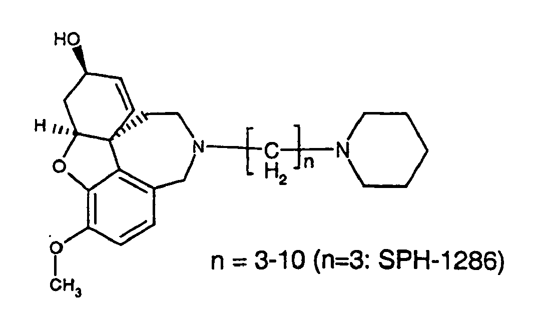

In gleicher Weise sind anstelle von Desoxypeganin auch dessen in der Literatur beschriebene Derivate zu verstehen, insoweit diese gleichzeitig Hemmstoffe der Acetylcholinesterase und von Monoaminooxidasen sind. Dazu zählen das in *Synthetic Communs.* 25(4), 569-572 (1995) beschriebene 7-Bromdesoxy-peganin, ebenso die in *Drug Des. Disc.* 14, 1-14 (1996) beschriebenen 7-Halo-6-hydroxy-5-methoxydesoxypeganine der allgemeinen Formel 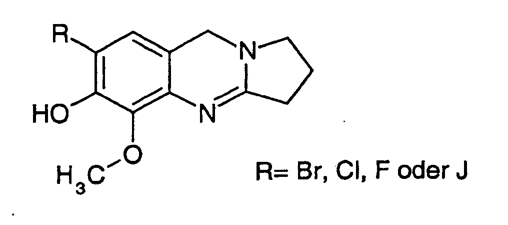
7-Brom-6-hydroxy-5-methoxydesoxypeganin
7-Chlor-6-hydroxy-5-methoxydesoxypeganin
7-Fluor-6-hydroxy-5-methoxydesoxypeganin
7-Jod-6-hydroxy-5-methoxydesoxypeganin

Des weiteren sind auch die in *Ind. J. Chem.* 24B, 789-790 (1985) beschriebenen Derivative des Desoxypeganins verwendbar, nämlich 1,2,3,9-Tetrahydro-6,7-methylenedioxypyrrolo[2,1-b]chinazolin und 2,3-Dihydro-6,7-dimethoxypyrrolo[2,1-b]chinazolin-9(1H)-on.

Die verabreichte Einzeldosis von Galanthamin oder einem seiner pharmakologisch akzeptablen Salze oder Derivate liegt vorzugsweise im Bereich von 1 bis 50 mg, wohingegen die verabreichte Einzeldosis von Desoxypeganin oder einem seiner pharmakologisch akzeptablen Salze oder Derivate vorzugsweise im Bereich von 10 bis 500 mg liegt.

Nach der Erfindung werden Galanthamin oder Desoxypeganin oder eines ihrer pharmakologisch akzeptablen Salze oder Derivate mit zumindest einer Substanz kombiniert, die antagonistische Wirkungen an Opioidrezpetoren entfaltet.

Besonders vorteilhaft wird die Aufgabe durch eine Kombination mit Vertretern bestimmter Opioidrezeptorantagonisten und pharmakologisch akzeptabler Verbindungen erfüllt. Dazu zählen vor allem 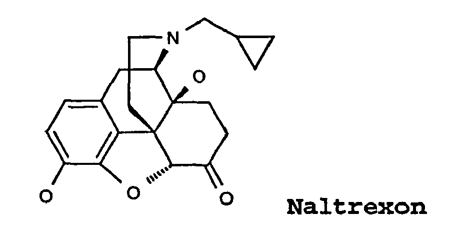 4,5-Epoxy-17-(cyclopropylmethyl)- 3,14-dihydroxymorphinan-6-on 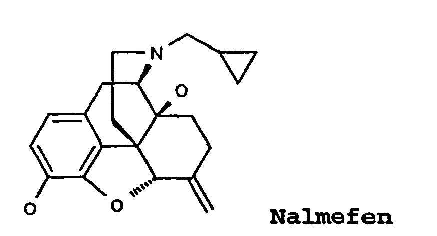 4,5-Epoxy-5alpha-17-(cyclopropylmethyl-6-methylen-morphinan-3,14-diol 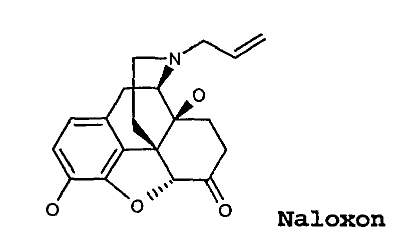 4,5-Epoxy-3,14-dihydroxy-17-(2-prophenyl)morphinan-6-on
sowie Nalorphin und Nalbuphin.

Es ist offensichtlich, dass diese Substanzen in Form aller ihrer pharmakologisch akzeptablen Salze und Additionsverbindugen angewendet werden können. So kann Naltrexon statt des meist verwendeten Hydrochlorides auch als Hydrobromid usw. eingesetzt werden. Ebenso ist offensichtlich, dass anstelle der oben angeführten Substanzen auch deren Derivate mit vergleichbarer pharmakologischer Wirksamkeit eingesetzt werden können, vor allem sämtliche in WO 0 112 196 (Southern Research Institute) beanspruchten, worunter sich insbesondere das folgende Derivat des Naltrexons befindet: 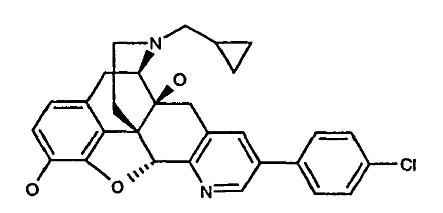 5'-(4-Chlorphenyl)-17-(cyclopropylmethyl)-6,7-didehydro-3,14-dihydroxy-4,5α-epoxypyrido[2',3':6,7]morphinan

Die verabreichte Einzeldosis von Naltrexon oder einem seiner pharmakologisch akzeptablen Salze oder Derivate liegt vorzugsweise im Bereich von 1 bis 200 mg.

Ebenso ist der Opioidrezeptormodulator Cyclazocin in seinen beiden stereoisomeren Formen ((+) und (-)) sowie als razemisches Gemisch einsetzbar, desgleichen Pentazocin. Die verabreichte Einzeldosis von Cyclazozin bzw. Pentazocin oder einem seiner pharmakologisch akzeptablen Salze oder Derivate liegt vorzugsweise im Bereich von 5 bis 100 mg.

Die Arzneiformen, welche gemäß vorliegender Erfindung zur Verabreichung einer Kombination von Modulatoren des cholinergen Systems mit einer als Opioidrezeptorantagonisten bzw. Opioidrezeptor-Modulator wirkenden Substanz verwendet werden, können einen oder mehrere folgender Zusatzstoffe enthalten:
- Antioxydantien, Synergisten, Stabilisatoren;
- Konservierungsmittel;
- Geschmackskorrigentien;
- Färbemittel;
- Lösemittel, Lösungsvermittler;
- Tenside (Emulgatoren, Solubilisatoren, Benetzer, Entschäumer);
- Viskositäts- und Konsistenzbeeinflusser, Gelbildner;
- Resorptionsbeschleuniger;
- Adsorptionsmittel, Feuchthaltemittel, Gleitmittel;
- Zerfalls- und Lösungsbeeinflusser, Füllmittel (Streckmittel), Peptisatoren;
- Freisetzungsverzögerer.

Diese Aufzählung ist nicht abschließend; die in Frage kommenden physiologisch unbedenklichen Substanzen sind dem Fachmann bekannt.

Die Verabreichung einer Kombination von Modulatoren des cholinergen Systems mit Opioidrezeptor-Antagonisten bzw. Modulatoren kann oral oder parenteral erfolgen. Für die orale Verabreichung können Arzneimittel in bekannten Darreichungsformen, wie Tabletten, Dragees oder Pastillen, hergestellt werden. Daneben kommen auch flüssige oder halbflüssige Darreichungsformen in Betracht, wobei der Wirkstoff als Lösung oder Suspension vorliegt. Als Löse- oder Suspendierungsmittel können Wasser, wässrige Medien oder pharmakologisch unbedenkliche Öle (vegetabilische oder Mineralöle) verwendet werden.

Vorzugsweise sind die eine Kombination von Modulatoren des cholinergen Systems mit einem Opioidrezeptor-Antagonisten bzw. Modulator enthaltenden Arzneimittel als Depot-Arzneimittel formuliert, welche in der Lage sind, diesen Wirkstoff über einen längeren Zeitraum in kontrollierter Weise an den Organismus abzugeben.

Darüber hinaus kann die Verabreichung einer Kombination von Modulatoren des cholinergen Systems mit einem Opioidrezeptor-Antagonisten bzw. Modulator nach der Erfindung auch auf parenteralem Wege erfolgen. Hierzu können transdermale oder transmucosale Darreichungsformen besonders vorteilhaft für die erfindungsgemäße Verabreichung einer Kombination von Modulatoren des cholinergen Systems mit einem Opioidrezeptor-Antagonisten verwendet werden, insbesondere klebende transdermale therapeutische Systeme (Wirkstoffpflaster). Diese ermöglichen es, den Wirkstoff über einen längeren Zeitraum in kontrollierter Weise über die Haut an den zu behandelnden Patienten abzugeben.

Ein weiterer Vorteil ist, daß eine mißbräuchliche Verwendung bei parenteralen Applikationsformen weniger leicht möglich ist als bei oralen Darreichungsformen. Durch die vorgegebene Wirkstoff-Freisetzungsfläche und die vorbestimmte Freisetzungsrate kann eine Überdosierung seitens des Patienten weitgehend ausgeschlossen werden. Außerdem sind transdermale Darreichungsformen aufgrund weiterer Eigenschaften sehr vorteilhaft, z. B. Vermeidung des First-Pass-Effektes oder eine bessere, gleichmässigere Steuerung des Blutspiegels.

Derartige transdermale, eine Kombination von Modulatoren des cholinergen Systems mit einem Opioidrezeptor-Antagonisten bzw. Modulatoren enthaltenden Systeme weisen üblicherweise eine wirkstoffhaltige, haftklebende Polymermatrix auf, die auf der hautfernen Seite von einer wirkstoffundurchlässigen Rückschicht bedeckt ist, und deren klebende, wirkstoffabgebende Oberfläche vor der Applikation mit einer ablösbaren Schutzschicht bedeckt ist. Die Herstellung solcher Systeme und die dabei verwendbaren Grundstoffe und Hilfsstoffe sind dem Fachmann grundsätzlich bekannt; beispielsweise wird der Aufbau solcher transdermalen therapeutischen Systeme in den deutschen Patenten DE 33 15 272 und DE 38 43 239 oder in den US-Patenten 4 769 028, 5 089 267, 3 742 951, 3 797 494, 3 996 934 und 4 031 894 beschrieben.

Die erfindungsgemäße Kombination eines Modulators des cholinergen Systems mit einem Opioidrezeptor-Antagonisten bzw. -Modulator kann bei der Nikotinentwöhnung verwendet werden, um den Konsum von Tabakprodukten zu verringern, insbesondere den von Zigaretten, jedoch auch von Kautabak.

In beispielhafter Weise wird die Aufgabe der Erfindung wie folgt gelöst, ohne daß der Umfang der Erfindung durch diese beispielhafte Aufzählung eingeschränkt werden soll.

### Beispiel 1

Oral oder transdermal zu verabreichendes Medikament, welches pro Einzeldosis 1 mg bis 50 mg Galanthamin in Form eines seiner pharmakologisch akzeptablen Salze, vorzugsweise in Form seines Hydrobromids, oder Additionsverbindungen und 10 bis 100 mg Naltrexon, vorzugsweise in Form des Hydrochlorids, enthält.

### Beispiel 2

Oral oder transdermal zu verabreichendes Medikament, welches pro Einzeldosis 10 mg bis 500 mg Desoxypeganin in Form eines seiner pharmakologisch akzeptablen Salze, vorzugsweise in Form seines Hydrochlorids, oder Additionsverbindungen und 10 bis 100 mg Naltrexon, vorzugsweise in Form des Hydrochlorids, enthält.

## Patentansprüche

1. Wirkstoff-Kombination aus zumindest einem Inhibitor der Cholinesterase, der auch auf dopaminerge Nervenendigungen wirkt, mit zumindest einem Opioidrezeptor-Antagonisten oder zumindest einer das Opioid-Rezeptorsystem modulierenden Substanz, welche an mu-Opioidrezeptoren antagonistisch und an kappa-Opioidrezeptoren agonistisch wirkt, zur medikamentösen Therapie der Nikotinabhängigkeit.

2. Wirkstoff-Kombination nach Anspruch 1, **dadurch gekennzeichnet, dass** der Inhibitor der Cholinesterase aus der Gruppe ausgewählt ist, die Galanthamin und Desoxypeganin in Form ihrer freie Base, ihrer Salze und Additions-verbindungen sowie deren pharmakologisch akzeptable Derivate umfasst.

3. Wirkstoff-Kombination nach Anspruch 1 oder 2, **dadurch gekennzeichnet, dass** der Opioidrezeptor-Antagonist oder zumindest einer der Opioidrezeptor-Antagonisten aus der Gruppe ausgewählt ist, die Naltrexon, Nalmefen, Naloxon, Nalorphin, Nalbuphin sowie deren pharmakologisch akzeptable Salze, Derivate und Additionsverbindungen umfasst.

4. Wirkstoff-Kombination nach Anspruch 3, **dadurch gekennzeichnet, dass** der Opioidrezeptor-Antagonist oder zumindest einer der Opioidrezeptor-Antagonisten vorzugsweise aus der Gruppe ausgewählt ist, die Naltrexon-Hydrochlorid, Naltrexon-Hydrobromid und 5'-(4-Chlorphenyl)-17-(cyclopropylmethyl)-6,7-didehydro-3,14-dihydroxy-4,5α-epoxypyridol-[2',3':6,7]morphinan umfasst.

5. Wirkstoff-Kombination nach Anspruch 1 oder 2, **dadurch gekennzeichnet, dass** die das Opioid-Rezeptorsystem modulierende Substanz oder zumindest eine der das Opioid-Rezeptorsystem modulierenden Substanzen aus der Gruppe ausgewählt ist, die Cyclazocin und Pentazocin in jeder ihrer beiden stereoisomeren Formen und als Gemisch sowie deren pharmakologisch akzeptable Salze und Derivate umfasst.

6. Wirkstoff-Kombination nach einem der vorhergehenden Ansprüche, **dadurch gekennzeichnet, dass** sie als Arzneiform vorliegt, wobei die zu verabreichende Einzeldosis von Galanthamin, seinen pharmakologisch akzeptablen Salzen, Additionsverbindungen oder Derivaten vorzugsweise in einem Bereich von 1 - 50 mg liegt, oder die zu verabreichende Einzeldosis von Desoxypeganin oder seinen pharmakologisch akzeptablen Salzen, Additionsverbindungen oder Derivaten vorzugsweise in einem Bereich von 10 - 500 mg liegt.

7. Wirkstoff-Kombination nach einem der vorhergehenden Ansprüche, **dadurch gekennzeichnet, dass** sie als Arzneiform vorliegt, wobei die zu verabreichende Einzeldosis von Naltrexon, seinen pharmakologisch akzeptablen Salzen, Additionsverbindungen oder Derivaten vorzugsweise in einem Bereich von 1 bis 200 mg liegt, oder die zu verabreichende Einzeldosis von Cyclazocin oder Pentazocin, ihren pharmakologisch akzeptablen Salzen oder Derivaten vorzugsweise in einem Bereich von 5 bis 100 mg liegt.

8. Wirkstoff-Kombination nach einem der vorhergehenden Ansprüche, **dadurch gekennzeichnet, dass** sie in Form einer Arzneiform vorliegt, die eine Depotwirkung aufweist.

9. Wirkstoff-Kombination nach einem der vorhergehenden Ansprüche, **dadurch gekennzeichnet, dass** sie in Form eines oral zu verabreichenden Arzneimittels vorliegt.

10. Wirkstoff-Kombination nach einem der vorangehenden Ansprüche, **dadurch gekennzeichnet, dass** sie in Form eines parenteral zu verabreichenden Arzneimittels vorliegt.

11. Wirkstoff-Kombination nach Anspruch 10, **dadurch gekennzeichnet, dass** sie in Form eines transdermal zu verabreichenden Arzneimittels vorliegt.

12. Verwendung einer Wirkstoff-Kombination nach einem der Ansprüche 1 bis 5 zur Herstellung einer Arzneiform zur medikamentösen Therapie der Nikotinabhängigkeit.

13. Verwendung nach Anspruch 12, **dadurch gekennzeichnet, dass** die Arzneiform in Form einer oralen Darreichungsform hergestellt wird.

14. Verwendung nach Anspruch 12, **dadurch gekennzeichnet, dass** die Arzneiform in Form einer parenteralen Darreichungsform hergestellt wird.

15. Verwendung nach Anspruch 14, **dadurch gekennzeichnet, dass** die Arzneiform in Form einer transdermalen Darreichungsform hergestellt wird.

16. Verwendung nach einem der Ansprüche 12 bis 15, **dadurch gekennzeichnet, dass** die Arzneiform eine zu verabreichende Einzeldosis von Galanthamin, seinen pharmakologisch akzeptablen Salzen, Additionsverbindungen oder Derivaten vorzugsweise in einem Bereich von 1 bis 50 mg, oder von Desoxypeganin, seinen pharmakologisch akzeptablen Salzen, Additionsverbindungen oder Derivaten vorzugsweise in einem Bereich von 10 bis 500 mg aufweist.

17. Verwendung nach einem der Ansprüche 12 bis 16, **dadurch gekennzeichnet, dass** die Arzneiform eine zu verabreichende Einzeldosis von Naltrexon, seinen pharmakologisch akzeptablen Salzen, Additionsverbindungen oder Derivaten vorzugsweise in einem Bereich von 1 bis 200 mg aufweist, oder von Cyclazocin bzw. Pentazocin oder ihren pharmakologisch akzeptablen Salzen oder Derivaten vorzugsweise in einem Bereich von 5 bis 100 mg aufweist.

## Claims

1. Active ingredient combination composed of at least one inhibitor of cholinesterase, which inhibitor also acts on dopaminergic nerve endings, with at least one opioid receptor antagonist or at least one substance modulating the opioid receptor system which has an antagonistic effect on mu opioid receptors and an agonistic effect on kappa opioid receptors, for the pharmacological therapy of nicotine dependence.

2. Active ingredient combination according to Claim 1, **characterized in that** the inhibitor of cholinesterase is selected from the group comprising galanthamine and deoxypeganine in the form of their free base, their salts and addition compounds and the pharmacologically acceptable derivatives thereof.

3. Active ingredient combination according to Claim 1 or 2, **characterized in that** the opioid receptor antagonist or at least one of the opioid receptor antagonists is selected from the group comprising naltrexone, nalmefene, naloxone, nalorphine, nalbuphine and the pharmacologically acceptable salts, derivatives and addition compounds thereof.

4. Active ingredient combination according to Claim 3, **characterized in that** the opioid receptor antagonist or at least one of the opioid receptor antagonists is preferably selected from the group comprising naltrexone hydrochloride, naltrexone hydrobromide and 5'-(4-chlorophenyl)-17-(cyclopropylmethyl)-6,7-didehydro-3,14-dihydroxy-4,5α-epoxypyrido[2',3':6,7]morphinan.

5. Active ingredient combination according to Claim 1 or 2, **characterized in that** the substance modulating the opioid receptor system or at least one of the substances modulating the opioid receptor system is selected from the group comprising cyclazocine and pentazocine in each of their two stereoisomeric forms and as mixture, and the pharmacologically acceptable salts and derivatives thereof.

6. Active ingredient combination according to any of the preceding claims, **characterized in that** it is in the form of a pharmaceutical form where the single dose of galanthamine, its pharmacologically acceptable salts, addition compounds or derivatives to be administered is preferably in a range from 1-50 mg, or the single dose of deoxypeganine or its pharmacologically acceptable salts, addition compounds or derivatives to be administered is preferably in a range from 10-500 mg.

7. Active ingredient combination according to any of the preceding claims, **characterized in that** it is in the form of a pharmaceutical form where the single dose of naltrexone, its pharmacologically acceptable salts, addition compounds or derivatives to be administered is preferably in a range from 1 to 200 mg, or the single dose of cyclazocine or pentazocine, their pharmacologically acceptable salts or derivatives to be administered is preferably in a range from 5 to 100 mg.

8. Active ingredient combination according to any of the preceding claims, **characterized in that** it is in the form of a pharmaceutical form which has a depot effect.

9. Active ingredient combination according to any of the preceding claims, **characterized in that** it is in the form of a medicament to be administered orally.

10. Active ingredient combination according to any of the preceding claims, **characterized in that** it is in the form of a medicament to be administered parenterally.

11. Active ingredient combination according to Claim 10, **characterized in that** it is in the form of a medicament to be administered transdermally.

12. Use of an active ingredient combination according to any of Claims 1 to 5 for producing a pharmaceutical form for pharmacological therapy of nicotine dependence.

13. Use according to Claim 12, **characterized in that** the pharmaceutical form is produced in the form of an oral dosage form.

14. Use according to Claim 12, **characterized in that** the pharmaceutical form is produced in the form of a parenteral dosage form.

15. Use according to Claim 14, **characterized in that** the pharmaceutical form is produced in the form of a transdermal dosage form.

16. Use according to any of Claims 12 to 15, **characterized in that** the pharmaceutical form comprises a single dose for administration of galanthamine, its pharmacologically acceptable salts, addition compounds or derivatives preferably in a range from 1 to 50 mg, or of deoxypeganine, its pharmacologically acceptable salts, addition compounds or derivatives preferably in a range from 10 to 500 mg.

17. Use according to any of Claims 12 to 16, **characterized in that** the pharmaceutical form comprises a single dose for administration of naltrexone, its pharmacologically acceptable salts, addition compounds or derivatives preferably in a range from 1 to 200 mg, or of cyclazocine or pentazocine or their pharmacologically acceptable salts or derivatives preferably in a range from 5 to 100 mg.

## Revendications

1. Combinaison de principes actifs, formée d'au moins un inhibiteur de la cholinestérase, qui agit sur les terminaisons nerveuses dopaminergiques, avec au moins un antagoniste de récepteur d'opioïde ou au moins une substance modulatrice du système récepteur d'opioïde, qui agit sur les récepteurs de muopioïdes de façon antagoniste et sur les récepteurs de kappa-opioïdes de façon agoniste, pour le traitement médicamenteux de la dépendance à la nicotine.

2. Combinaison de principes actifs selon la revendication 1, **caractérisée en ce que** l'inhibiteur de la cholinestérase est choisi dans le groupe comprenant la galanthamine et la désoxypéganine sous leur forme de base libre, de sel et de composé d'addition, et leurs dérivés pharmacologiquement acceptables.

3. Combinaison de principes actifs selon la revendication 1 ou 2, **caractérisée en ce que** l'antagoniste de récepteur d'opioïde, ou au moins un des antagonistes de récepteur d'opioïde, est choisi dans le groupe comprenant la naltrexone, le nalméfène, la naloxone, la nalorphine, la nalbuphine ainsi que leurs sels, dérivés et composés d'addition pharmacologiquement acceptables.

4. Combinaison de principes actifs selon la revendication 3, **caractérisée en ce que** l'antagoniste de récepteur d'opioïde, ou au moins un des antagonistes de récepteur d'opioïde, est choisi de préférence dans le groupe comprenant le chlorhydrate de naltrexone, le bromhydrate de naltrexone et le 5'-(4-chlorophényl)-17-(cyclopropylméthyl)-6,7-didéhydro-3,14-dihydroxy-4,5a-époxypyridol-[2',3':6,7]-morphinane.

5. Combinaison de principes actifs selon la revendication 1 ou 2, **caractérisée en ce que** la substance modulatrice du système récepteur d'opioïde ou au moins une des substances modulatrices du système récepteur d'opioïde est choisi dans le groupe comprenant la cyclazocine et la pentazocine sous la forme de leurs deux stéréoisomères ou de mélanges de ces derniers, ainsi que leurs sels et dérivés pharmacologiquement acceptables.

6. Combinaison de principes actifs selon l'une quelconque des revendications précédentes, **caractérisée en ce qu'**elle se présente sous forme de médicament dans lequel la dose unitaire de galanthamine, de ses sels, dérivés ou composés d'addition pharmacologiquement acceptables à administrer est comprise de préférence dans la gamme de 1-50 mg, ou la dose unitaire de désoxypéganine ou de ses sels, dérivés et composés d'addition pharmacologiquement acceptables à administrer est comprise de préférence dans la gamme de 10 - 500 mg.

7. Combinaison de principes actifs selon l'une quelconque des revendications précédentes, **caractérisée en ce qu'**elle se présente sous forme de médicament dans lequel la dose unitaire de naltrexone, de ses sels, dérivés ou composés d'addition pharmacologiquement acceptables à administrer est comprise de préférence dans la gamme de 1 à 200 mg, ou la dose unitaire de cyclazocine ou de pentazocine, de ses sels et dérivés pharmacologiquement acceptables à administrer est comprise dans la gamme de 5 à 100 mg.

8. Combinaison de principes actifs selon l'une quelconque des revendications précédentes, **caractérisée en ce qu'**elle se présente sous forme de médicament retard.

9. Combinaison de principes actifs selon l'une quelconque des revendications précédentes, **caractérisée en ce qu'**elle se présente sous forme de médicament à administrer par voie orale.

10. Combinaison de principes actifs selon l'une quelconque des revendications précédentes, **caractérisée en ce qu'**elle se présente sous forme de médicament à administrer par voie parentérale.

11. Combinaison de principes actifs selon la revendication 10, **caractérisée en ce qu'**elle se présente sous forme de médicament à administrer par voie transdermique.

12. Utilisation d'une combinaison de principes actifs selon l'une quelconque des revendications 1 à 5, pour la préparation d'un médicament destiné au traitement de la dépendance à la nicotine.

13. Utilisation selon la revendication 12, **caractérisée en ce que** la forme médicamenteuse est préparée sous une forme destinée à l'administration par voie orale.

14. Utilisation selon la revendication 12, **caractérisée en ce que** la forme médicamenteuse est préparée sous une forme destinée à l'administration par voie parentérale.

15. Utilisation selon la revendication 14, **caractérisée en ce que** la forme médicamenteuse est préparée sous une forme destinée à l'administration par voie transdermique.

16. Utilisation selon l'une quelconque des revendications 12 à 15, **caractérisée en ce que** la forme médicamenteuse comprend une dose unitaire à administrer, contenant de la galanthamine ou ses sels, dérivés et composés d'addition pharmacologiquement acceptables, qui est comprise de préférence dans la gamme de 1 à 50 mg, ou une dose unitaire à administrer, contenant de la désoxypéganine ou ses sels, dérivés et composés d'addition pharmacologiquement acceptables, comprise de préférence dans la gamme de 10 à 500 mg.

17. Utilisation selon l'une quelconque des revendications 12 à 16, **caractérisée en ce que** la forme médicamenteuse comprend une dose unitaire à administrer, contenant du naltrexone, ou ses sels, dérivés et composés d'addition pharmacologiquement acceptables, qui est comprise de préférence dans la gamme de 1 à 200 mg, ou une dose unitaire à administrer, contenant de la cyclazocine ou de la pentazocine ou leurs sels et dérivés pharmacologiquement acceptables, qui est comprise de préférence dans la gamme de 5 à 100 mg.
